Europäisches Patentamt
European Patent Office
Office européen des brevets

(19)

(11) Publication number: 0 266 057 A2

# EUROPEAN PATENT APPLICATION

(12)

(21) Application number: 87308484.2

(22) Date of filing: 25.09.87

(51) Int. Cl.4: C12N 15/00 , C12N 5/00 , C12P 21/02

The title of the invention has been amended (Guidelines for Examination in the EPO, A-III, 7.3).

(30) Priority: 01.10.86 US 913909

(43) Date of publication of application: 04.05.88 Bulletin 88/18

(84) Designated Contracting States: CH DE FR GB IT LI NL

(71) Applicant: MERCK & CO. INC.
126, East Lincoln Avenue P.O. Box 2000
Rahway New Jersey 07065-0900(US)

(72) Inventor: Bayne, Marvin L.
131 Tudor Oval
Westfield New Jersey 07090(US)
Inventor: Kopchick, John J.
119 Morningside Road
Verona New Jersey 07044(US)

(74) Representative: Hesketh, Alan, Dr.
European Patent Department Merck & Co., Inc. Terlings Park Eastwick Road
Harlow Essex, CM20 2QR(GB)

## (54) Plasmid containing an eucaryotic secretory signal sequence and process for secreting encoded proteins.

(57) A synthetic gene encoding an exogenous protein has been assembled and inserted into a eucaryotic secretion expression vector containing the cytomegalovirus immediate early (CMV-IE) transcriptional regulatory region, any one of a variety of mammalian secretory signal peptides and a portion of the bovine growth hormone gene encoding transcription termination sequences. The recombinant plasmid encodes the amino acid for the fusion protein that contains the mammalian secretory signal peptide and the amino acids of the protein to be secreted. This plasmid, when transiently introduced into cultured mammalian fibroblasts, directs the synthesis of the fusion protein, the subsequent proteolytic removal of the secretory signal peptide and the secretion of the protein into the culture media. The recombinant proteins produced by these cells are biologically active.

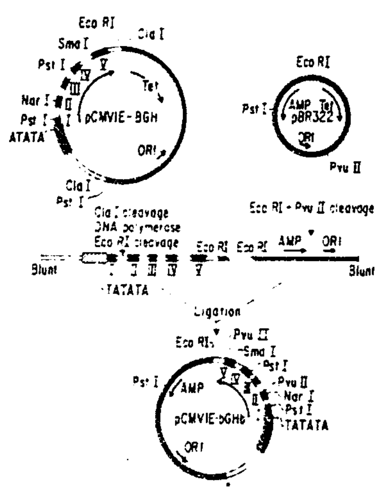


FIG. 1

Xerox Copy Centre

EP 0 266 057 A2

# SYNTHETIC GENES ENCODING PROTEINS FROM PLASMID CONTAINING EUCARYOTIC SECRETORY SIGNAL PEPTIDES AND PROCESS FOR SECRETING ENCODED PROTEINS

## BACKGROUND OF THE INVENTION

The instant invention describes the expression of proteins in mammalian cells. Previous reports have described the expression of a mammalian protein such as human insulin growth factor (hIGF-I) in bacteria (See Buell et al., Nucleic Acids Research , 13 pg 1923- (1985); Peters et al., Gene 35 pg 83-89 (1984); and Nilsson et al., Nucleic Acids Research 13, pg 1151- (1985)). Human IGF-I is produced in E. coli in a denatured inactive form that must be renatured in vitro in order to produce biologically active proteins (See Peters et al. supra and Horlein et al., Program and Abstracts of the 67th Annual Meeting of the Endocrine Society , pg 63, Abstract 250 (1985)). Such manipulations would complicate the interpretation of biological activity of the recombinant protein, especially of structural analogs. Synthesis of a staphylococcal protein A-hIGF-I fusion protein in Staphylococcus aureus results in the secretion of a non-denatured protein, but this procedure requires chemical cleavage to remove the Staph A protein region from the fusion protein (See Nilsson et al supra). Alternatively, expression of mammalian growth factors in yeast using α-factor hybrid gene fusions allows the secretion of biologically active proteins. See Brake et al., Proc. Nat. Acad. Sci., USA 81 pg. 4642-4646 (1984). The instant procedure yields the desired protein directly from mammalian cell culture fluids without the need for additional biological or chemical procedures to renature the protein or to remove secretory signal peptides.

## SUMMARY OF THE INVENTION

The instant invention concerns a plasmid derived from the cytomegalovirus immediate early promoter (CMV-IE) into which an exogenous protein gene encoding eucaryotic proteins has been inserted and optionally into which a mammalian secretory signal peptide has been incorporated. When this plasmid is introduced into mammalian cells and these cells cultured, the protein is secreted into the medium in a biologically active form. Thus it is an object of this invention to describe the preparation of the p-CMV-IE with the exogenous protein gene incorporated therein. A further object is to describe the preparation of the plasmid with the mammalian secretory signal peptide incorporated therein. It is a further object to describe the preparation and incorporation into the p-CMV-IE of the gene for the mammalian protein which is to be secreted by cultured cells, preferably mammalian cells, with the recombinant plasmid incorporated therein. Further objects will become apparent from the reading of the following description.

## DESCRIPTION OF THE INVENTION

Recombinant DNA molecules in which a promoter regulatory element from the human cytomegalovirus immediate early region has been ligated to an exogenous gene are described herein. Using a transient eucaryotic expression assay system, plasmid constructs were screened for their ability to direct expression and secretion of the protein or peptide coded for by the exogenous gene. These constructs were co-transformed into a cell culture, preferably a mammalian, such as a mouse, cell culture, in order to obtain a stable cell culture secreting large amounts of the protein or peptide coded for by the exogenous gene.

The preparation of a plasmid containing the cytomegalovirus immediate early promoter with the bovine growth hormone gene incorporated therein has been described. See Pasleau et al., Gene 38 pg 227-232 (1985). This plasmid transformed into cultured mammalian fibroblasts directs the synthesis and secretion of bovine growth hormone. The instant invention contemplates the recombinant transformation of this plasmid in two ways. First, the plasmid can be constructed with a synthetic gene, cDNA clones, genomic clones or a combination thereof which, when the plasmid is transformed into cultured mammalian cells, a mammalian protein corresponding to the synthetic gene is secreted by the cells. Second, the plasmid may be constructed with the synthetic gene incorporated therein and the bGH secretory signal peptide may be substituted by the secretory signal peptide of another mammalian protein. The doubly modified plasmid will still express and secrete the mammalian protein into the cell culture medium in biologically active form without the need for any additional biological or chemical procedures.

The exogenous genes which can be incorporated into the p-CMV-IEbGHb plasmid to be expressed by the transformed cultured mammalian cells are those derived from human growth hormone, human somatocrinin, human placental lactogen, human proinsulin, human insulin-like growth factor 1, human insulin-like growth factor 2, human complement factor 5A, human blood coagulation factors, human $\alpha$-interferon A, B, C, D, E, or F, human $\beta$-interferon, human $\gamma$-interferon, human interleukin-1 or -2, human apolipoprotein A-1, human antithrombin III, human prosomatostatin, human proenkephalin A or B, bovine parathyroid hormone, bovine growth hormone, bovine vasopressin, bovine proalbumin, canine amylase, canine proinsulin, hamster proinsulin and the like.

The secretory signal peptides which may also be incorporated into the CMV-IE plasmid to express the mammalian protein also constructed into the plasmid are described for the above proteins and many others in Watson, Nucleic Acids Research 12, pg 5145-5164 (1984).

The cytomegalovirus immediate early promoter could be replaced by the following eucaryotic transcriptional regulatory sequences: mouse metallothionein, mouse mammary tumor virus long terminal repeat, simian virus 40 early or late promoters, herpes thymidine kinase, rous sarcoma virus long terminal repeat, Moloney leukemia virus long terminal repeat or any eucaryotic transcriptional regulatory sequence.

Attached hereto are five figures which further describe and explain the instant invention.

Figure 1 describes the preparation of recombinant plasmid pCMVIE-bGHb from plasmid pCMVIE-BGH and plasmid pBR322 by selective cleavage and recombination. This plasmid encodes for the secretory secretory signal peptide of bovine growth hormone or any other secretory signal peptide substituted in this plasmid in the region marked I and II.

Figure 2. Sequence of the synthetic h-IGF-I gene. The numbers identify the 22 oligonucleotides used in the gene assembly. The lines denote the ends of the oligonucleotides and the respective overlapping regions between fragments. Unique restriction endonuclease cleavage sites are marked by arrows. Other genes described above could be synthesized or cloned in a similar vector.

Figure 3. pCMVIE-bGH-b contains the ampicillin resistance gene (AMP) and DNA origin of replication (ORI) derived from pBR322 (hatched lines), exons I through V (black boxes) derived from the bovine growth hormone gene, and the CMV-IE promoter (cross hatching). The "TATA" box within the CMV-IE promoter is indicated. phIGF, a pBR322 derivative (hatched lines) contains the chemically synthesized human IGF-I gene inserted between the NdeI and Sal I restriction endonuclease cleavage sites. The construction of pCMV-bGH-hIGF is described in the Examples. This ampicillin resistant plasmid contains exons I and portions of exons II which encodes the bGH secretory signal peptide and V of the bGH gene (black boxes) ligated to the hIGF-I coding sequences.

Figure 4. Western gel analysis of transiently transformed mouse L-cells. Cell culture supernatants were analyzed by 15% SDS-PAGE and immunoblotted with anti-hIGF-I antiserum. Lane A, 50 ng of $Thr^{59}$-hIGF-I; lane B, supernatant from untransformed cells; lane C, supernatant from cells transformed with pCMVIE-bGH-b; lane D, supernatant from cells transfected with pCMV-bGH-hIGF.

Figure 5. Stimulation of DNA synthesis in A10 cells by pCMV-bGH-hIGF transfected L cell conditioned media. Cells were incubated in serum free media (SFM) in the absence or presence of IGF-I (50 nM), conditioned media (CM) from L cells or conditioned media from transiently transfected L cells. The amount of $^3H$-thymidine incorporation into DNA was estimated as described in the Example Data are expressed as the mean ± standard deviation of three determinations.

Plasmid pCMVIE-BGH or any similar plasmid with a mammalian secretory signal peptide in place cf the bovine growth hormone secretory signal peptide is subjected to Cla I cleavage and EcoRI cleavage and the plasmid pBR322 is subject to EcoRI + PvuII cleavage to obtain two fragments with the secretory signal peptide coding region contained therein. Ligation of the two fragments produces the plasmid pCMVIE-bGHb which has the coding region for bovine growth hormone and a region coding for a mammalian secretory signal peptide inserted in the region identified as I and II in Figure 1.

The exogenous protein gene is preferably a mammalian protein gene. The ends of the purified exogenous protein gene are modified to allow for ligation in proper translational framing to the NarI and SmaI ends of pCMVIE-bGHb.

The plasmid pCMVIE-bGHb supplemented with the secretory signal peptide at I and II is subjected to NarI and SmaI or PvuII cleavage to produce a fragment with the majority of the gene for bovine growth hormone excised and the mammalian secretory signal peptide present. The two fragments are ligated to produce a plasmid containing both the secretory signal peptide and mammalian protein genes. The general procedure used for the cleavage and ligation of plasmids and fragments are described in Molecular Cloning --A Laboratory Manual, T. Maniatis et al.

The proteins secreted by the mammalian cells into the culture medium are in a biologically active form and require no further processing to be fully utilized.

The instant invention is further described in the following example which should not be considered limitation of the instant invention.

## EXAMPLE

### Reagents and Enzymes

Restriction endonucleases were purchased from New England Biolabs. T4 DNA ligase and T4 polynucleotide kinase and nucleic acid grade agarose were obtained from Pharmacia, $Thr^{59}$-IGF-I analog and antisera against it were from Amgen. $^{125}I$-Protein A was obtained form Amersham. Elutip-d columns were from Schleicher and Schuell. Sep-Pak $C_{18}$ cartridges were purchased from Waters Associates. Diisopropyl phosphoramidites were obtained from Applied Biosystems. Phosphotriester reagents were from Biosearch. $^{3}H$-thymidine (20 Ci/mmol) was purchased from Amersham Corp. Purified human IGF-I was from Dr. R.E. Humbel, $\gamma$-$^{32}P$-ATP was from Amersham. Nu-serum was obtained-from Hyclone. 2,6-diaminopurine was from Sigma.

### Synthesis and Purification of Oligodeoxynucleotides

Oligodeoxynucleotides 1-9, and 12-22 were synthesized using phosphotriester chemistry on a Biosearch Model Sam 1 oligonucleotide synthesizer. Oligodeoxynucleotides 10 and 11 were synthesized by phosphoramidite chemistry on an Applied Biosystems Model 380A synthesizer. Oligodeoxynucleotides 1GF-101 and 1GF-102 were synthesized by phosphoramidite chemistry on the Biosearch synthesizer. Oligonucleotides were purified by 12% or 20% polyacrylamide gel electrophoresis under denaturing conditions or by high performance liquid chromatography using a PEI column and a linear 15% acetonitrile in $H_2O$ to a 15% acetonitrile in 1M $NH_4SO_4$ gradient. Gel purified oligonucleotides were eluted from the gel in 200 mM triethylammonium bicarbonate and purified on Sep-Pak $C_{18}$cartridges.

### Phosphorylation and Ligation of Oligodeoxynucleotides

Oligodeoxynucleotides used in the assembly of the synthetic hIGF-I gene were phosphorylated as follows: 1 mmole of each purified oligonucleotide was incubated in a 40 ul solution of 20 mM Tris-HCl pH 7.5, 10 mM $MgCl_2$, 2 mM EDTA, 5 mM DTT, 50 um ($\gamma$-$^{32}P$-ATP) (specific activity 5 Ci/mMole) containing 10 units of T4 polynucleotide kinase. After 1 hour at 37°C the kinase was inactivated by heating to 95°C for 5 minutes. Indicated subsets of fragments were mixed, heated to 95°C for 5 minutes then slow cooled to 25°C over 4 hours to allow the complementary strands to anneal. The ATP concentration was then adjusted to 1 mM, T4 DNA ligase was added to 0.8 units/10 ul, and the reaction mixture incubated overnight at 15°C. Sections, A, B and C were purified on a 7 M urea, 12% polyacrylamide gel run in 90 mM Tris-borate, 2 mM EDTA. DNA fragments were eluted from the gel and purified on Sep-Pak $C_{18}$ cartridges using triethylammonium bicarbonate as described above.

### Plasmid Construction and DNA Cloning

Standard procedures for plasmid purification (Birnboim et al ., Nucleic Acid Research 7, pg 1513- (1979)) transformation (Mandel et al., J. Mol. Biol. 53, pg 154- (1970)) and DNA sequencing (Maxam et al., Methods Enzymol 65, pg 499-560 (1980)) were used. Large plasmid fragments were purified by agarose gel electrophoresis followed by electrolution and purification on elutip-d columns. Small DNA fragments (<800 bp) were purified by polyacrylamide gel electrophoresis followed by elution in ammonium acetate and purification on elutip-d columns.

Cell Culture and Transient Expression Assays

Mouse L cell fibroblasts (thymidine kinase negative and adenine phosphoribosyl transferase negative) were maintained in Dulbecco's Modified Eagle's Medium (DMEM) containing 20% Nu serum and 50 ug/ml 2,6 diaminopurine. The transient mouse L cell expression assay system using DEAE-Dextran was previously described (Kopchick et al., DNA 4 , pg 23 et seq (1985)). For Western gel analysis 200 ul of cell culture supernatant was concentrated to 40 ul and analyzed on a 15% acrylamide $NaDodSO_4$ gel. Following separation on the gel the proteins were transferred to nitrocellulose filters. The filters were then treated with 0.5% gelatin in PBS for 1 hour prior to incubation with a 1:100 dilution of anti-IGF antiserum followed by $^{125}$I-labeled protein A. Autoradiographs were prepared using Kodak XAR film.

Radioimmunoassay

Culture fluids were assayed for hIGF-I by a standard double antibody radioimmunoassay procedure. Results obtained are expressed as ng/ml using $Thr^{59}$-analog of hIGF-I as standard.

$^{125}$I-IGF-I Binding to Human Placental Membranes

$^{125}$I-IGF-I (specific activity, 85 Ci/g) was prepared as previously described (18). Freshly obtained placenta was homogenized in 0.25 M sucrose containing 25 mM benzamidine HCL, 5 mM EDTA, and 0.2 mM PMSF using a blender at 4°C. The homogenate was centrifuged at 10,000 $^{\times}$g for 30 minutes. The supernatant was adjusted to give 0.1 M NaCl and 0.2 mM $MgCl_2$ final concentration and was centrifuged at 100,000 $^{\times}$g for 1 hour. The 100,000 $^{\times}$g pellet was resuspended in 10 mM Tris-HCl, pH 7.5 containing 0.15 M NaCl by homogenization and centrifuged as above. The final pellet was suspended at 12 mg/ml protein in assay buffer and stored at -70°C. $^{125}$I-IGF-I (50 fmol) membranes (0.2 mg) and 20-50 ul tissue culture supernatant were incubated at 20°C for 1 hour in 0.2 ml 0.1 M Hepes, pH 8 containing 120 mM NaCl, 5 mM KCl, 0.12 mM $MgSO_4$ and 0.1% BSA. Samples were filtered over Whatman GF/F filters presoaked in 0.1% polyethyleneimine to separate bound from free ligand. The incubation tubes and filters were washed 4 times with 2.5 ml with cold assay buffer (minus BSA).

$^{125}$I-IGF-I Binding to Acid Stable Serum Binding Proteins

Freshly prepared human serum (18 ml) was incubated with 1 M acetic acid for 3 hours at 22°C, then centrifuged at 10,000 $^{\times}$g for 20 minutes. The supernatant was applied to a Sephadex G-50 column (3 cm $^{\times}$ 85 cm) and eluted in 1 M acetic acid. The void volume protein peak was pooled and dialyzed for 24 hours against $H_2O$ at 4°C then lyophilized. The crude binding proteins were reconstituted in phosphate buffered saline pH 7.5 containing 2 mg/ml BSA, and stored at -70°C at a protein concentration of 15-20 mg/ml.

$^{125}$I-IGF-I (50 fmol) was incubated with 0.1 mg crude binding protein and 2-5 $\mu$l of tissue culture supernatant in 0.15 ml phosphate buffered saline, pH 7.5 containing 0.2% BSA for 24 hours at 4°C. The assay was terminated by the addition of 1 ml of 5% Norit A charcoal in 2% BSA. After 30 minutes at 4°C, samples were centrifuged in an Eppendorf microfuge for 20 minutes at 4°C. An aliquot (0.9 ml) of the supernatant was removed and counted.

$^{3}$H-thymidine Incorporation Into DNA

The measurement of $^{3}$H-thymidine incorporation into DNA in the cloned vascular smooth muscle cell line, A10 was performed as described previously (20). Briefly, cells were grown to confluence in 16 mm wells, washed two times with 1 ml DMEM/0.1% BSA, then incubated in 1 ml DMEM/0.1% BSA for 36 hours at 37°C. Cells were incubated with tissue culture supernatants in 0.5 ml DMEM/0.1% BSA for 8 hours at 37°C before the addition of 1 uCi/ml H-thymidine (20 Ci/mmol). After an additional 16 hours at 37°C, the cells were washed 4 times with 1 ml phosphate buffered saline, then lysed by the addition of 0.3 ml of 0.4 N NaOH. The lysates were supplemented with calf thymus DNA (0.2 mg), then neutralized with 0.3 ml of 0.4 N HCl. DNA was precipitated by the addition of 1.2 ml absolute ethanol (-20°C).

After 2 hours at -20°C, the precipitates were collected on Whatman GF/F filters, and wells and filters were washed 5 times with 2 ml ice cold 70% ethanol. Filters were cut and incubated with 10 ml Aquasol II (New England Nuclear Corp.) for 2 hours before counting.

## Construction of the Synthetic Gene

A synthetic gene encoding the 70 amino acids of hIGF-I was designed based on the known amino acid sequence. The nucleotide sequence for the gene was selected by optimizing the number of unique restriction endonuclease recognition sites, and by avoiding secondary structure problems and poorly utilized E. coli codons. Twenty-two oligonucleotides, ranging in size from 10 to 40 nucleotides, were synthesized, purified and assembled to form the synthetic hIFG-I gene (Figure 2).

To assemble the gene the oligonucleotides were divided into three sections; Section A, oligo's 1-9; section B, oligo's 10-15; and section C, oligo's 16-22. The 5′ termini of all oligonucleotides with the exception of numbers 1 and 22 were phosphorylated using T4 polynucleotide kinase then annealed and ligated. Sections A, B and C were purified by polyacrylamide gel electrophoresis under denaturing conditions, annealed and ligated to form the intact gene. The synthetic gene was ligated to Nde I/Sal I digested pBR322 and transformed into E. coli HB101. A clone containing the predicted DNA sequence, as determined by Maxam et al sequencing, was designated pBR-hIGF.

## Construction of Expression Vector

Plasmid pCMV-IE-bGH is a pBR322 based expression plasmid containing the immediate early transcriptional regulatory region of cytomegalovirus (CMV-IE) joined to the bovine growth hormone (bGH) gene. This plasmid transformed into cultured mouse fibroblasts directs the synthesis and secretion of bGH. During the secretion process the secretory signal peptide of bGH is removed. A derivative of this plasmid, pCMV-IE-bGH-b was constructed by moving the EcoRI, Cla I expression cassette from pCMV-IE-bGH into EcoRI, Pvu II digested pBR322 (see Figure 3). This plasmid has a unique Nar I site in exon II of the bGH gene at the junction of the signal peptide and the secreted form of bGH. In order to insert the synthetic hIGF-I gene into the Nar I site of pCMV-IE-bGH-b, two oligonucleotides were synthesized;
IGF-101 5′ CGCCGGACCGGAGACGCTCTGCGGGGCTGAGCT 3′, and IGF-102 5′ CAGCCCCG-CAGAGCGTCTCCGGTCCGG 3′. These oligonucleotides were annealed to form a Nar I/Sac I fragment that regenerates the first ten amino acids of hIGF-I. This manipulation allows the synthetic hIGF-I gene to be joined to the bGH signal peptide at the Nar I Site. The modified hIGF-I gene was inserted between the Nar I and Sma I sites of pCMV-IE-bGH-b (Figure 2). The resulting plasmid, pCMV-bGH-hIGF encodes a 97 amino acid fusion protein consisting of the 26 amino acid bGH signal peptide, the first amino acid (Ala) of bGH and the 70 amino acids of hIGF-I. The amino acid sequence at the junction of the bGH signal peptide and hIGF-I is the following:

```
_______bGH Signal____     _____hIGF-I_____
Trp-Thr-Gln-Val-Val-Gly-Ala-Gly-Pro-Glu-Thr
```

The expression vector retains the 3′ end of the bovine growth hormone gene that contains the polyadenylation signal as well as putative transcriptional termination signals. DNA sequence analysis of pCMV-bGH-IGF confirmed that the predicted hIGF-I coding sequence was inserted.

## Expression of hIGF-I

Plasmids pCMVIE-bGH-b and pCMV-bGH-hIGF were introduced transiently into mouse L cell fibroblasts by the DNA-DEAE-Dextran sulfate technique. Three days later the cells were rinsed three times in serum-free media then incubated an additional 40 hours in serum-free media. Aliquots of the cell culture media were analyzed for the presence of hIGF-I by radioimmunoassay, radioreceptor assay using human placental membranes, and by binding to acid stable human IGF serum carrier proteins. No detectable amount of IGF-I is measured by radioimmunoassay or by binding to acid stable human IGF carrier proteins in conditioned media from L cells alone, or from L cells transfected with the pCMVIE-bGH-b vector. Media from cells

transfected with pCMV-bGH-hIGF contain 110 or 190 ng/ml IGF-I-like activity when measured by radioimmunoassay or serum carrier protein binding activity, respectively (Table I). Small amounts of receptor active IGF-I-like material are measured in L cell conditioned media and in media from cells transfected with pCMVIE-bGH-b (Table II). There is a 7-14 fold increase in the level of IGF-I measured by radioreceptor assay in cells transfected with pCMV-bGH-hIGF (Table I). Since no IGF-I like activity is detectable in the control media in the other two assays the apparent receptor binding activity in the controls may be due to an unrelated contaminant in conditioned media which interferes in the receptor assay. A likely candidate for this interfering contaminant is the tissue-derived IGF binding protein described by DeVroede et al (J. Clin. Invest. 77, pg 602-613 (1986)) and Clemmons et al (J. Clin. Invest. 77, pg 1548-1556 (1986)).

To further characterize the hIGF-I produced by the transiently transformed mouse fibroblasts, the cell culture media was analyzed by SDS-polyacrylamide gel electrophoresis followed by immunoblotting using anti-hIGF-I antiserum. As shown in Figure 4, cells transformed by pCMV-bGH-hIGF (lane D) secrete a protein identical in size to the $Thr^{59}$ analog of hIGF-I (lane A). No IGF-I like material is seen in media derived from cells transformed with pCMV-bGH-b, or from normal mouse L cells conditioned media (lanes B and C). The size of the recombinant hIGF-I suggests that the bGH signal peptide has been removed during the secretion process. Amino acid sequencing of hIGF-I produced by mouse L cells stably transformed with pCMV-bGH-hIGF. Amino acid sequencing of hIGF-I produced by mouse L cells stably transformed with pCMV-bGH-hIGF yields the sequence Ala-Gly-Pro-Glu. . . etc. indicating that the alanine residue at the junction of the bGH secretory signal peptide and the synthetic hIGF-I gene remains at the amino terminus of the recombinant hIGF-I.

## Biological Activity

It has been previously shown that IGF-I stimulates DNA synthesis in rat A10 vascular smooth muscle cells (Cascieri et al., Circ. Res. pg (1986)). We used this assay to measure the biological activity of our recombinant hIGF-I. Figure 5 demonstrates the activity of conditioned media from cells transiently transformed with pCMV-bGH-hIGF. IGF-I (50 nM) causes a 2 fold increase in the incorporation of $^3H$-thymidine into DNA of A10 cells. Conditioned media from pCMV-bGH-hIGF transfected L cells, which contained 20 nM receptor active IGF-I, causes a 2.5 fold increase in DNA synthesis when compared to non-transfected L cells conditioned media (Figure 5).

It has been shown previously that a chimeric plasmid containing the cytomegalovirus immediate early (CMV-IE) transcriptional regulatory region fused to the bovine growth hormone (bGH) gene will direct synthesis and secretion of bGH by transiently transformed mouse L cells (Pasleau et al., Gene 38, pg 227-232 (1985)). This plasmid, pCMV-IE-bGH contains transcriptional initiation signals derived from the CMV genome and transcriptional termination signals of the bGH gene. The plasmid also encodes a 26 amino acid signal peptide believed to be involved in bGH secretion. In the plasmid pCMV-IE-bGH-b, a unique Nar I restriction enzyme cleavage site occurs in exon II of the bGH gene at the junction between the bGH signal peptide and the mature protein (Figure 3). We have inserted a synthetic human IGF-I gene at this site to test if the bGH signal peptide can direct secretion of a heterologous protein by transfected mouse L cells. Following the transient introduction of the plasmid pCMV-bGH-hIGF into cultured mouse cells, biologically active hIGF-I is found in the culture fluid (Table I and Figure 5). The generation of mouse L cell lines stably transformed with the expression plasmid has allowed purification of biologically active recombinant hIGF-I.

This study suggests that the plasmid pCMV-bGH-b may be a useful expression vector for the synthesis and secretion of heterologous proteins in cultured mammalian cells. Mammalian cell culture may be the expression system of choice when specific post-translational modifications such as glycosylation, phosphorylation or amidation are required to produce recombinant proteins with full spectrums of biological activities.

TABLE I

Human IGF-I Expression By
Transiently Transformed Mouse L-Cells

| DNA | Human IGF-I (ng/ml) R.I.A. | R.R.A. | S.B.P. |
|---|---|---|---|
| None | N.D. | 92 | N.D. |
| pCMV-IE-bGH-b | N.D. | 51 | N.D. |
| pCMV-bGH-hIGF | 110 | 730 | 190 |

Mouse L-cells were transiently transformed with either plasmid pCMV-IE-bGH-b or pCMV-bGH-hIGF-I. Cell culture supernatants were assayed for hIGF-I by radioimmunoassay (RIA), radioreceptor assay (RRA) or binding to serum binding proteins (SBP) as described above. N.D. indicates that no activity above background was detected in these assays.

## Claims

1. A plasmid pCMV-bGHb with a bovine growth hormone secretory signal peptide and the gene encoding bovine growth hormone replaced by an exogenous protein gene encoding eucaryotic proteins.
2. The plasmid of Claim 1 wherein the exogenous protein gene is a mammalian protein gene.
3. A plasmid pCMV-bGHb of Claim 1 further modified by replacing the bovine growth hormone secretory signal peptide with a different mammalian secretory signal peptide.
4. The plasmid of Claim 1 where the cytomegalovirus immediate early promoter region is replaced by a different eucaryotic transcriptional regulatory sequence.
5. Eucaryotic fibroblasts incorporating the plasmid of Claim 1.
6. Eucaryotic fibroblasts incorporating the plasmid of Claim 3.
7. A process for the preparation of the plasmid of Claim 1 which comprises:
   (a) cleavage of plasmid pCMV-IE-BGH with the appropriate restriction endonuclease such that the secretory signal peptide for bovine growth hormone is replaced by another mammalian secretory signal peptide;
   (b) cleavage of plasmid pBR322 at EcoRI and PvuII;
   (c) ligation of the fragment obtained from steps a) and b).
8. A process for the preparation of the plasmid of Claim 3 which comprises:
   (a) cleavage of plasmid pCMV-IE-BGH, or plasmid pCMV-IE-BGH with the secretory signal peptide for bovine growth hormone replaced by another mammalian secretory signal peptide, at Cla I and EcoRI;
   (b) cleavage of plasmid pBR322 at EcoRI and Pvu II;
   (c) ligation of the fragments obtained from steps a) and b);
   (d) cleavage of the plasmid obtained from Step c) at NarI and SmaI, or NarI and PvuII;
   (e) modification of purified eucaryotic protein gene to allow for ligation between the NarI and SmaI or NarI and PvuII;
   (f) ligation of the fragments obtained from steps d) and e).
9. A process for the preparation of exogenous proteins which comprises cultivating the fibroblasts of Claim 11 and isolating the excreted exogenous protein.

10. The h-IGF-I gene:

5′ T ATG GGT CCG GAA ACT CTG TGT GGC GCC GAG CTC GTT AC CCA GGC CTT TGA GAC ACA CCG CGG CTC GAG CAA

GAC GCT CTG CAG TTC GTT TGC GGT GAC CGC GGT TTC CTG CGA GAC GTC AAG CAA ACG CCA CTG GCG CCA AAG

TAC TTC AAC AAA CCG ACT GGT TAC GGT TCT TCT TCT ATG AAG TTG TTT GGC TGA CCA ATG CCA AGA AGA AGA

AGA CGT GCT CCG CAG ACT GGT ATC GTT GAT GAA TGC TCT GCA CGA GGC GTC TGA CCA TAG CAA CTA CTT ACG

TGC TTC AGA TCT TGT GAC CTG CGT CGT CTC GAG ATG ACG AAG TCT AGA ACA CTG GAC GCA GCA GAG CTC TAC

TAC TGC GCA CCG CTG AAA CCG GCT AAA TCT GCT TGA ATG ACG CGT GGC GAC TTT GGC CGA TTT AGA CGA ACT

TAA G
ATT CAGCT 5′

Neu eingereicht / Newly filed
Nouvellement déposé



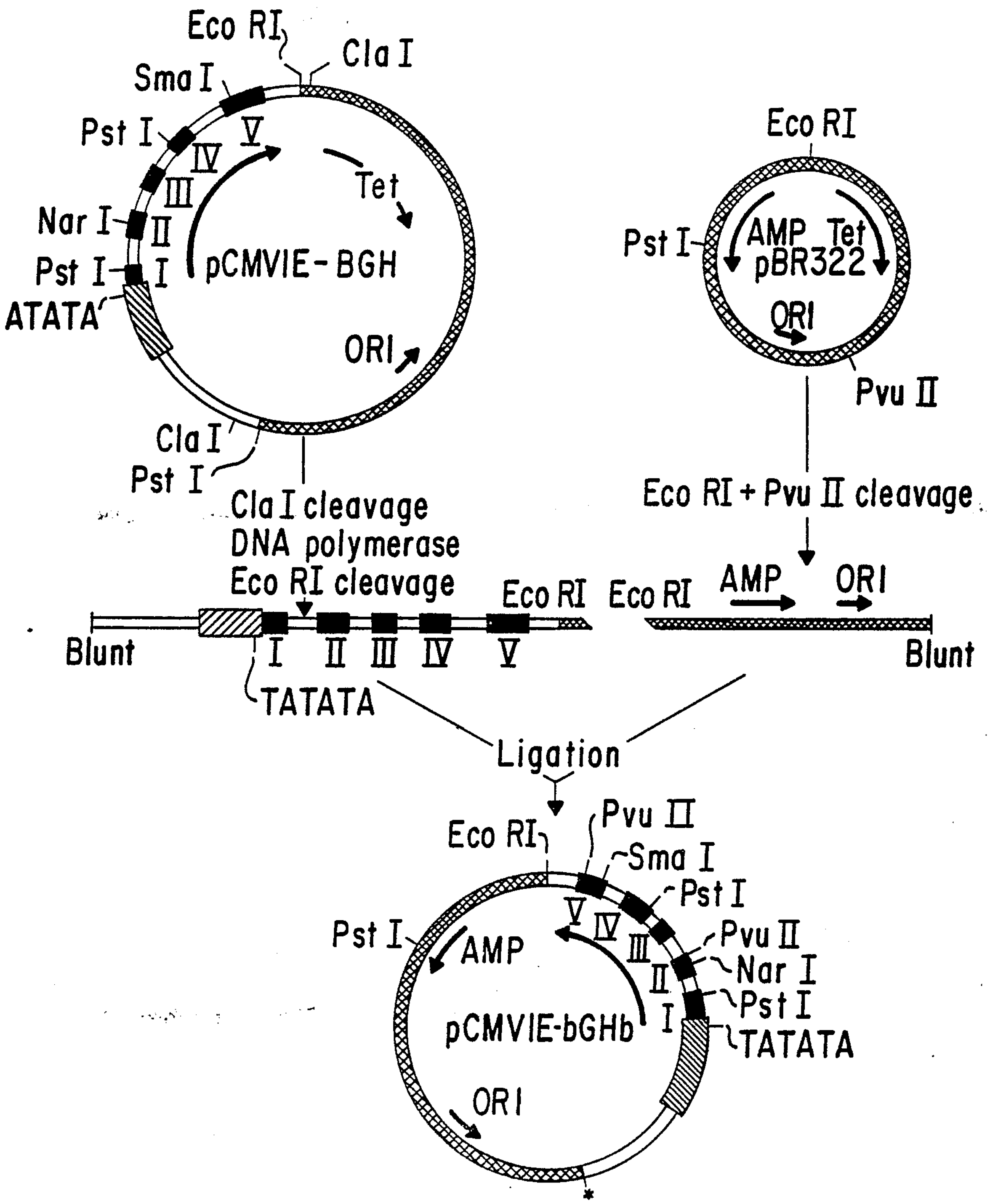


FIG. 1

# FIG. 2A

(1) (3) (5)

Met Gly Pro Glu Thr Leu Cys Gly Ala Glu Leu Val Asp Ala Leu Gln Phe Val Cys

5' T ATG GGT CCG GAA ACT CTG TGT GGC GCC GAG CTC GTT GAC GCT CTG CAG TTC GTT TGC

AC CCA GGC CTT TGA GAC ACA CCG CGG CTC GAG CAA CTG CGA GAC GTC AAG CAA ACG

(2) (4) (6)

Sac I Pst I

(7) (9) (11)

Gly Asp Arg Gly Phe Tyr Phe Asn Lys Pro Thr Gly Tyr Gly Ser Ser Ser Arg Arg

GGT GAC CGC GGT TTC TAC TTC AAC AAA CCG ACT GGT TAC GGT TCT TCT TCT AGA CGT

CCA CTG GCG CCA AAG ATG AAG TTG TTT GGC TGA CCA ATG CCA AGA AGA AGA TCT GCA

(8) (10)

BstE II Xba I

FIG. 2B

(13) (15)

Ala Pro Gln Thr Gly Ile Val Asp Glu Cys Cys Phe Arg Ser Cys Asp Leu Arg Arg

GCT CCG CAG ACT GGT ATC GTT GAT GAA TGC TGC TTC AGA TCT TGT GAC CTG CGT CGT

CGA GGC GTC TGA CCA TAG CAA CTA CTT ACG ACG AAG TCT AGA ACA CTG GAC GCA GCA

(12) (14) (16)

Bgl II

(17) (19) (21)

Leu Glu Met Tyr Cys Ala Pro Leu Lys Pro Ala Lys Ser Ala * *

CTC GAG ATG TAC TGC GCA CCG CTG AAA CCG GCT AAA TCT GCT TGA TAA G

GAG CTC TAC ATG ACG CGT GGC GAC TTT GGC CGA TTT AGA CGA ACT ATT CAGCT 5'

(18) (20) (22)

Xho I

Neu eingereicht / Newly filed
Nouvellement déposé



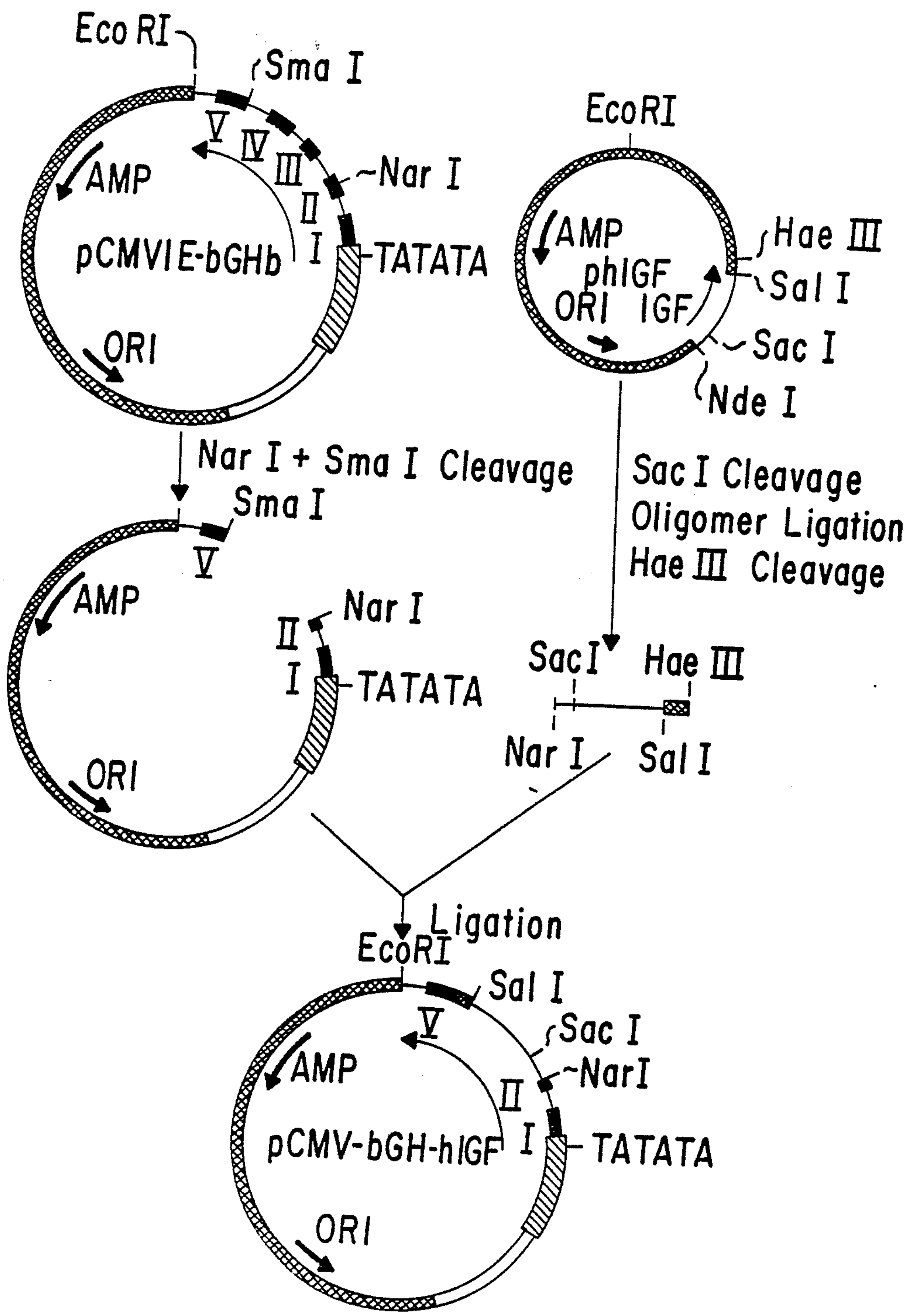


FIG. 3

FIG. 4

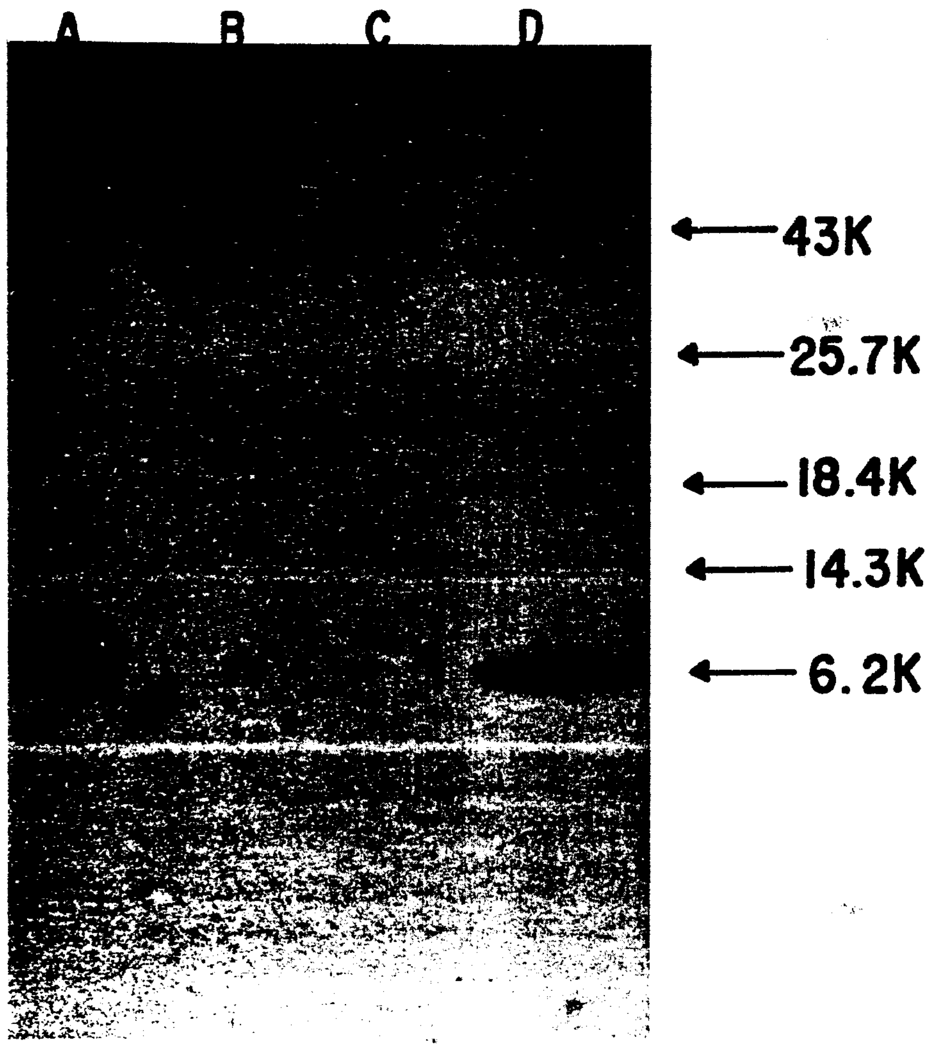

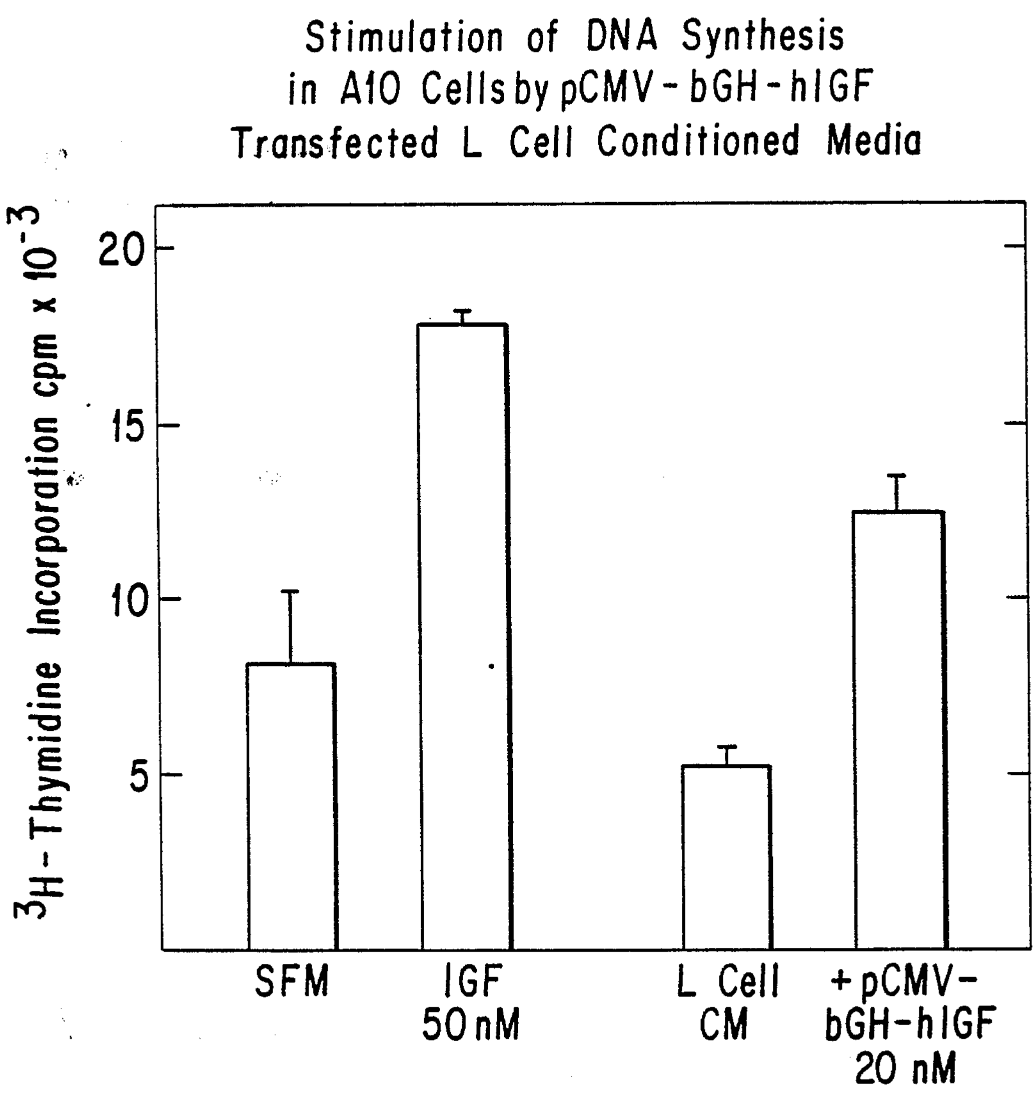

Stimulation of DNA Synthesis
in A10 Cells by pCMV-bGH-hIGF
Transfected L Cell Conditioned Media
3H-Thymidine Incorporation cpm x 10-3
20
15
10
5
SFM
IGF 50 nM
L Cell CM
+pCMV-bGH-hIGF 20 nM


FIG. 5